# EUROPÄISCHE PATENTSCHRIFT

(11) **EP 1 974 703 B1**
(45) Veröffentlichungstag und Bekanntmachung des Hinweises auf die Patenterteilung: **20.01.2010**
(21) Anmeldenummer: 07005598.3
(22) Anmeldetag: 19.03.2007
(51) Int. Cl.: A61F 9/01

(54) **Augenschonendes Lasersystem für die refraktive Chirurgie**
Laser system for refractive conservative surgery for the eye
Système laser embelliseur d'yeux pour la chirurgie réfractive

(43) Veröffentlichungstag der Anmeldung: 01.10.2008
(73) Patentinhaber: WaveLight AG, 91058 Erlangen (DE)
(72) Erfinder: Donitzky, Christof, 90542 Eschenau (DE)
(74) Vertreter: von Hellfeld, Axel

(56) Entgegenhaltungen:
- WO-A-89/06519
- US-A- 5 348 551
- US-A- 5 656 186
- US-A- 6 146 375
- US-A1- 2004 176 752
- US-A1- 2005 149 006
- US-B1- 6 258 082

## Beschreibung

Die Erfindung betrifft ein augenschonendes Lasersystem für die refraktive Chirurgie.

In der Ophthalmologie wird unter "refraktiver Chirurgie" mit Lasern die Wechselwirkung von Laserstrahlung mit Teilen des Auges verstanden, um die brechenden Eigenschaften des Auges und damit seine Abbildungseigenschaften zur Beseitigung oder zumindest Linderung von Abbildungsfehlern zu ändern.

Ein besonders bedeutsames Beispiel der refraktiven Chirurgie ist die Korrektur der Fehlsichtigkeit eines Auges mit der LASIK-Technik. Bei der LASIK gemäß dem Stand der Technik wird zunächst die Hornhaut mittels z.B. eines Mikrokeratoms seitlich aufgeschnitten und das so entstehende Deckelchen (auch Flap genannt) wird zur Seite geklappt. Im so freigelegten Stroma der Hornhaut (Kornea) erfolgt mit Laserstrahlung eine sogenannte Ablation, d.h. Entfernung von Gewebe gemäß einem berechneten Ablationsprofil. Danach wird das Deckelchen zurückgeklappt und es erfolgt ein relativ schmerzfreier und schneller Heilungsprozess. Nach diesem Eingriff hat die Kornea andere Abbildungseigenschaften und die Fehlsichtigkeit ist behoben oder gemindert.

Üblicherweise erfolgt der oben beschriebene seitliche Einschnitt (Inzision) in die Kornea beim Stand der Technik mit einem sogenannten Mikrokeratom, d.h. einer oszillierenden mechanischen Schneide. In jüngerer Zeit wurden auch sogenannte Femtosekunden-Mikrokeratome eingesetzt, bei denen Femtosekunden-Laserimpulse im Gewebe der Hornhaut fokussiert werden, um dort mit eng benachbarten Brennpunkten der Strahlung einen sogenannten laserinduzierten Durchbruch oder sogenannte laserinduzierte Photodisruptionen im Hornhautgewebe zu erzeugen, die so im Hornhautgewebe durchgeführt werden, dass letztlich ein Schnitt wie bei einem mechanischen Mikrokeratom entsteht.

Je nach Art der Bearbeitung (z.B. Inzision oder Ablation) und/oder Gewebetyp wird in der laseroptischen Augenchirurgie Laserstrahlung unterschiedlicher Wellenlängen und/oder Impulsdauern eingesetzt. Für die Anbringung von Schnitten (Inzision) in der Hornhaut (etwa für die Präparation eines Flaps) ist es üblich, Laserstrahlung im Bereich von etwa 340 - 350 nm oder im nahen infraroten (NIR) Wellenlängenbereich, beispielsweise zwischen 1000 und 1100 nm, mit einer Impulsdauer im Femtosekundenbereich einzusetzen. Ein derartiges System wird auch als Femtosekunden-Mikrokeratom bezeichnet. Dagegen wird für die Photoablation von Stromagewebe in der Regel auf Laserstrahlung im ultravioletten Wellenlängenbereich, beispielsweise 193 nm, zurückgegriffen, wobei die verwendete Impulsdauer auch länger sein kann, bis hin in den Nanosekundenbereich.

Im Allgemeinen tritt beim Schnitt des Flaps mit dem Femtosekundenlaser eine Energietransmission von etwa 40% durch die Hornhaut auf. Diese durch die Hornhaut weitergeleitete Energie kann im Auge zu einer starken Strahlenbelastung führen, die sich beim Patienten als Nebenwirkung, beispielsweise als sogenanntes Transient Light Syndrom (TLS), über mehrere Monate negativ äußert.

Wird beim Schnitt des Flaps mit einem Femtosekundenlaser eine sichtbare Wellenlänge verwendet, beispielsweise ein Titan-Saphir-Laser mit einer Wellenlänge von 710 nm bis 810 nm oder ein frequenzverdoppeltes Infrarotsystem mit einer Wellenlänge von etwa 517 nm, tritt eine nicht hinnehmbare visuelle Belastung des Patienten während des Operationsprozesses auf.

UV-Femtosekundenlasersysteme mit einer Frequenzverdreifachung von infraroter Strahlung geben eine Wellenlänge von etwa 345 nm ab, indem beispielsweise die dritte Oberwelle erzeugt wird. Bei dieser Wellenlänge ergibt sich eine extrem effiziente Umsetzung der Laserstrahlenergie beim Photodisruptionsprozess. Dennoch treten etwa 5 % der Energie weiter in das Auge ein und werden in der Linse absorbiert. Ferner entsteht eine Blaulicht-Fluoreszenz mit einem Fluoreszenzmaximum bei 440 nm, was der Spitze des sogenannten Blue-Light-Hazard-Effekts (Blaulichtgefährdung) entspricht und hauptsächlich eine Schädigung der Retina (Netzhaut) nach sich zieht.

Die WO 89/06519 A2 offenbart die Verwendung einer Wellenlänge im Bereich von 1400 bis 1800 nm bei der intrastromalen Keratomeleosis, um die Krümmung der Kornea zu modifizieren.

Die US 2004/0176752 schlägt vor, mittels eines Cr:YAG-Lasers Augengewebe zu schmelzen.

Die US 5,348,551 offenbart die Verwendung eines Co:Mg:F₂-Lasers bei einem Fehlsichtigkeitskorrekturverfahren, bei dem Keratozyten zwischen den Lamellen der Cornea verletzt oder beschädigt werden, um die Corneakrümmung zu ändern. Bei diesem Verfahren wird im Gegensatz zu photorefraktiven Verfahren das Kollagen im Behandlunqsbereich nicht geschrumpft oder geschädigt.

Die US 6,258,082 B1 offenbart einen Diodenlaser mit einer Wellenlänge von 980 nm, 1,5 µm und 1,9 µm sowie einen diodengepumpten Er:YAG-Laser mit einer Wellenlänge von etwa 2,94 µm. Die von diesem Laser abgegebene Strahlung wird bei der photorefraktiven Keratektomie, der phototherapeutischen Keratektomie, der intrastromalen Photokeratektomie, der LASIK und der LASE angewendet.

Die US 5,656,186 beschäftigt sich mit der Ablation und einem laserinduzierten Durchbruch der Kornea mit Laserimpulsen vom Bereich von 150 fsec bis 7 nsec. Als Wellenlänge werden hierfür 770 nm verwendet.

Die Veröffentlichung "Generation of 20-fs pulses by a prismless Cr4+:YAG laser", Ripin et alteri, Optics Letter, Vol. 27, No. 1, 1 January 2002 offenbart einen Cr⁴⁺:YAG-Laser, bei dem Impulse kürzer als 20 fsec bei einem prismafreien Laser gemessen wurde. Die Impulse hatten ihr Maximum bei 1450 nm, und in einem Bereich von 1310 nm bis 1500 nm war die Intensität höher als die Hälfte des Maximums. Mittels eines logarithmischen Maßstabs konnte ein Spektrum von 1140 nm bis 1700 nm, was die Grenze des verwendeten Spektralmessgerätes war, beobachtet werden.

Es ist eine Aufgabe der Erfindung, ein augenschonendes Augenbehandlungsgerät zu schaffen.

Die Aufgabe wird durch ein Augenbehandlungsgerät mit einer Laserstrahlungsquelle gemäß Anspruch 1 gelöst. Das von der Strahlungsquelle abgegebene Licht weist einen derartigen Wellenlängenbereich auf, dass es in einem Behandlungsbereich eines Auges eine Reaktion bewirkt und in zumindest einem der in Richtung Netzhaut dahinter liegenden Bereiche zumindest teilweise absorbiert wird. Dies hat den Vorteil, dass die durch den Behandlungsbereich hindurchtretende Lichtstrahlung absorbiert wird und sich Schädigungen der hinter dem Behandlungsbereich liegenden Strukturen vermeiden lassen.

Das von der Strahlungsquelle abgegebene Licht kann einen Wellenlängenbereich aufweisen, in dem der Behandlungsbereich teilweise durchlässig ist.

Der Behandlungsbereich ist die Hornhaut. Die durch die Hornhaut hindurchtretende Lichtstrahlung kann beispielsweise im Kammerwasser absorbiert werden. Folglich lassen sich Schäden an hinter dem Kammerwasser liegenden Strukturen, beispielsweise der Iris, der Linse, dem Glaskörper, der Retina, vermeiden.

Die durch das Licht im Behandlungsbereich bewirkte Reaktion kann eine Ablation von Gewebe sein. Durch die Ablation von Gewebe kann die Hornhaut neu geformt werden, um eine eventuelle Fehlsichtigkeit zu korrigieren. Die durch das Licht im Behandlungsbereich bewirkte Reaktion ist ein laserinduzierter optischer Durchbruch von Gewebe der auch als Photodisruption bezeichnet wird. Mit Hilfe der laserinduzierten Durchbrüche bzw. der Photodisruptionen wird ein Schnitt in der Hornhaut erzeugt.

Die Strahlungsquelle ist eine Laserquelle. Zum Erzeugen der laserinduzierten Durchbrüche wird eine Femtosekunden-Laserquelle verwendet.

Der Wellenlängenbereich des von der Strahlungsquelle abgegeben Lichts beträgt etwa 1600 nm bis etwa 1700 nm, vorzugsweise etwa 1625 nm bis etwa 1675 nm, höchst bevorzugt etwa 1640 nm bis etwa 1660 nm. In diesen Wellenlängenbereichen ist die Hornhaut lichtdurchlässig und das durch die Hornhaut hindurchtretende Licht wird im Kammerwasser absorbiert, wodurch eine Schädigung der hinter dem Kammerwasser liegenden Strukturen, beispielsweise der Iris, der Linse, des Glaskörpers, oder der Retina, vermeiden werden kann. Als Femtosekunden-Lasersysteme für den Wellenlängenbereich 1600 - 1700 nm kommen insbesondere in Betracht die Systeme Co:MgF₂ und Cr:YAG.

Ein Verfahren zur Behandlung des Auges mit Licht weist einen derartigen Wellenlängenbereich auf, dass es in einem Behandlungsbereich eine Reaktion bewirkt und in zumindest einem der in Richtung Netzhaut dahinter liegenden Bereiche zumindest teilweise absorbiert wird. Dies hat den Vorteil, dass die aus dem Behandlungsbereich heraustretende Lichtstrahlung absorbiert wird und sich Schädigungen der hinter dem Behandlungsbereich liegenden Strukturen vermeiden lassen.

Der Behandlungsbereich kann in dem Wellenlängenbereich im Wesentlichen durchlässig sein. Der Behandlungsbereich ist die Hornhaut . Die durch die Hornhaut hindurchtretende Lichtstrahlung kann, wie zuvor erwähnt wurde, beispielsweise im Kammerwasser absorbiert werden. Folglich lassen sich Schäden an hinter dem Kammerwasser liegenden Strukturen, beispielsweise der Iris, der Linse, dem Glaskörper, der Retina, vermeiden.

Die durch das Licht im Behandlungsbereich bewirkte Reaktion kann eine Ablation von Gewebe sein. Die durch das Licht im Behandlungsbereich bewirkte Reaktion kann auch ein laserinduzierter optischer Durchbruch von Gewebe sein. Die Strahlungsquelle ist eine Laserquelle .

Der Wellenlängenbereich beträgt bei dem Verfahren etwa 1600 nm bis etwa 1700 nm, vorzugsweise etwa 1625 nm bis etwa 1675 nm, höchst bevorzugt etwa 1640 nm bis etwa 1660 nm. Wie zuvor erwähnt wurde, ist in diesen Wellenlängenbereichen die Hornhaut lichtdurchlässig und das durch die Hornhaut hindurchtretende Licht wird im Kammerwasser absorbiert, wodurch eine Schädigung der hinter dem Kammerwasser liegenden Strukturen, beispielsweise der Iris, der Linse, des Glaskörpers, der Retina, vermeiden werden kann.

Die Erfindung wir nun mit Hilfe der beigefügten Figuren detaillierter beschrieben.
- Figur 1: zeigt ein Augenmodell nach Gullstrand - Le Grand,
- Figur 2: ist ein Diagramm, das die Transmission der Hornhaut darstellt und
- Figur 3: ist ein Diagramm, das die Transmission des Kammerwassers darstellt.

Dem Fachmann auf dem Gebiet der Ophthalmologie, insbesondere der refraktiven Chirurgie, sind zur Ablation und Inzision geeignete Strahlungsquellen bekannt. Diese umfassen Laserlichtquellen. Wie eingangs erwähnt wurde, werden zur Inzision Impulsdauern im Femtosekundenbereich und für die Ablation längere Impulsdauern verwendet. Zur Anpassung der Wellenlänge des Lasers auf die Anwendung können sogenannte Frequenzvervielfacher verwendet werden. Derartige Lasersysteme sind dem Fachmann bekannt und müssen nicht näher beschrieben werden.

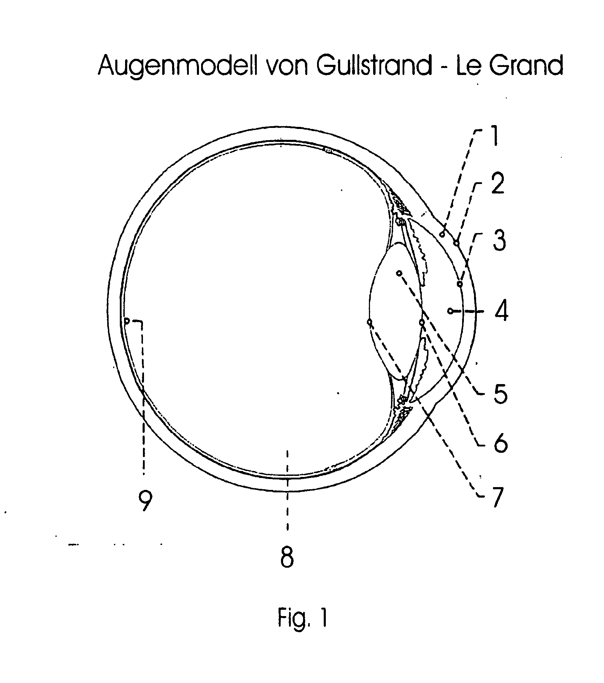

Figur 1 zeigt ein Augenmodell von Gullstrand - Le Grand. Die Hornhaut (Cornea) 1 hat eine vordere Oberfläche 2 und eine hintere Oberfläche 3. Hinter der hinteren Oberfläche 3 der Hornhaut 1 befindet sich das Kammerwasser (Aqueous) 4. Hinter dem Kammerwasser 4 befindet sich die Linse 5 mit einer vorderen Oberfläche 6 und einer hinteren Oberfläche 7. Hinter der Linse schließt sich der Glaskörper (Vitereous) 8 an. Hinter dem Glaskörper 8 befindet sich die Netzhaut (Retina) 9. Das Licht fällt durch die Cornea 1 in das Auge und wird auf die Netzhaut 9 abgebildet.

Wie eingangs erwähnt wurde, wird bei der refraktiven Chirurgie mittels eines Femtosekundenlasers durch einen laserinduzierten optischen Durchbruch ein Schnitt in der Hornhaut 1 erzeugt. Es versteht sich, dass die Laserstrahlung durch den laserinduzierten Durchbruch in der Hornhaut 1 nicht vollständig absorbiert wird. Bei Augenbehandlungsgeräten des Standes der Technik passiert der nicht in der Hornhaut 1 absorbierte Teil der Laserstrahlung die Kammerflüssigkeit 4, die Linse 5 und den Glaskörper 8 und trifft auf der Netzhaut 9 auf. Je nach verwendeter Wellenlänge können beim Patienten Nebenwirkungen auftreten, beispielsweise das zuvor erwähnte Transient Light Syndrom oder eine Schädigung der Netzhaut 9 aufgrund des Blue-Light-Hazard-Effekts.

Auch bei der Ablation von Gewebe in der Hornhaut 1 zum Korrigieren einer Fehlsichtigkeit kann bei Augenbehandlungsgeräten des Standes der Technik der nicht in der Hornhaut 1 absorbierte Teil der Laserstrahlung die Kammerflüssigkeit 4, die Linse 5 und den Glaskörper 8 passieren und auf der Netzhaut 9 auftreffen. Auch hierbei entstehen je nach verwendeter Wellenlänge die zuvor beschriebenen Nebenwirkungen. Die Strahlung des Excimerlasers mit einer Wellenlänge von 193 nm wird hingegen in der Hornhaut vollständig absorbiert.

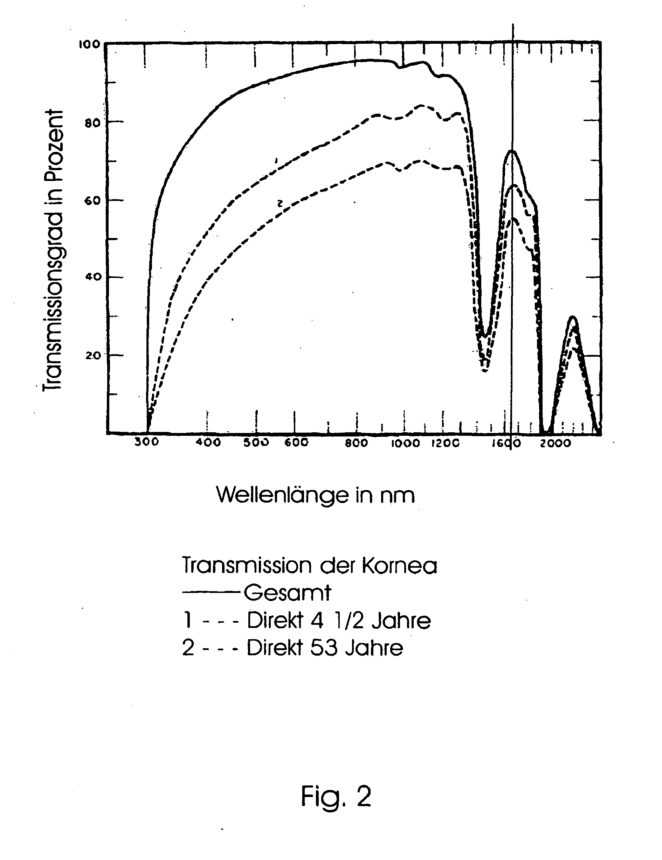

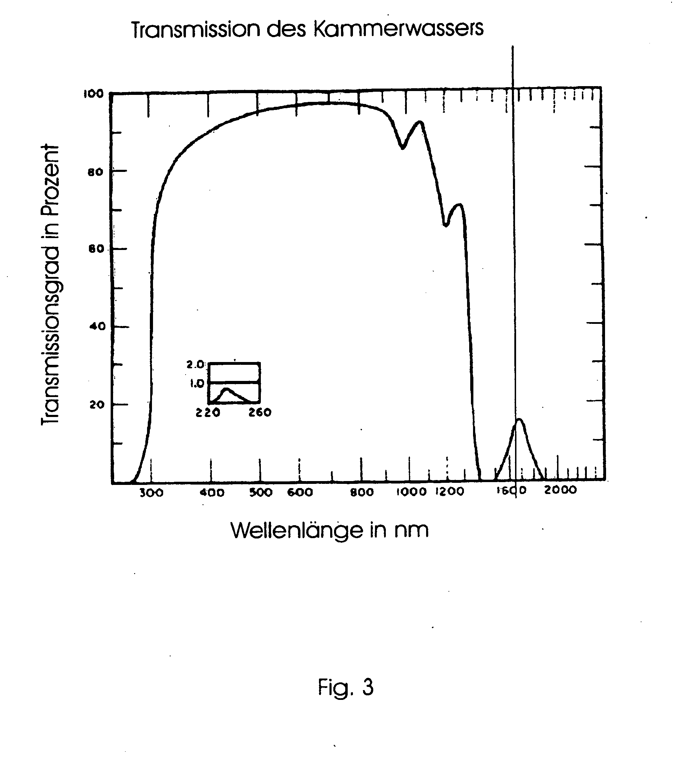

Figur 2 ist ein Diagramm, das den Transmissionsgrad der Hornhaut darstellt. Figur 3 ist ein Diagramm, das den Transmissionsgrad des Kammerwassers darstellt. In Figur 1 zeigt die durchgezogene Linie die Gesamttransmission der Hornhaut 1, die mit sechs Augen ermittelt wurde. Die in Figur 1 mit 1 bezeichnete Kurve zeigt die direkte Transmission bei einem Auge eines 4 ½ Jahre alten Kindes, und die mit 2 bezeichnete Kurve zeigt die direkte Transmission bei einem Auge eines 53 Jahre alten Menschen.

Sowohl bei der Inzision als auch der Ablation ist die Hornhaut der zuvor genannte Behandlungsbereich. Eine Behandlung soll nicht nur an der Oberfläche der Hornhaut 1, sondern auch in tieferen Bereichen der Hornhaut 1 möglich sein. Folglich soll der zur Behandlung ausgewählte Wellenlängenbereich derart ausgewählt werden, dass die Hornhaut 1 in diesem Wellenlängenbereich teilweise durchlässig ist. Dazu bietet sich gemäß Figur 2 zum einen der Wellenlängenbereich von 300 nm bis 1300 nm und der Wellenlängenbereich von 1600 nm bis 1700 nm an.

Es wurde erkannt, dass sich die zuvor beschriebenen Nebenwirkungen vermeiden lassen, wenn die nicht im Behandlungsbereich, d. h. der Hornhaut, absorbierte Strahlung in einem dahinter liegenden Bereich absorbiert wird. In diesem Fall kann die nicht in der Hornhaut absorbierte Strahlung beispielsweise nicht die Linse und/oder die Netzhaut 9 erreichen.

Es wird vorgeschlagen, zur Behandlung der Hornhaut 1 einen Wellenlängenbereich von etwa 1600 nm bis etwa 1700 nm zu verwenden, da in diesem Bereich das Kammerwasser 4 eine vergleichsweise niedrige Transmission aufweist. Folglich findet die Absorption der nicht in der Hornhaut 1 absorbierten Strahlung in einem Bereich statt, der möglichst nahe an der Hornhaut 1 liegt. Wird die nicht in der Hornhaut 1 absorbierte Strahlung im Kammerwasser 4 absorbiert, kann sie nicht oder nur um die Absorption im Kammerwasser 4 geschwächt die Iris, die Linse 5 und den Glaskörper 8 passieren und auf der Netzhaut 9 auftreffen. Dadurch werden eine Schädigung oder eine Beeinträchtigung anderer Bereiche des Auges und die zuvor beschriebenen Nebenwirkungen weitgehend vermieden.

## Patentansprüche

1. Hornhaut-Flap-Schnittgerät das einen Schnitt in der Hornhaut (1) erzeugt, mit einer Laserstrahlungsquelle, die Laserimpulse im Femtosekundenbereich zum Erzeugen eines Schnitts in der Hornhaut (1) abgibt, wobei sich das von der Laserstrahlungsquelle abgegebene Licht in einem derartigen Wellenlängenbereich befindet, dass es in der Hornhaut (1) eines Auges eine Reaktion bewirkt,
wobei die durch das Licht in der Hornhaut (1) bewirkte Reaktion ein laserinduzierter optischer Durchbruch von Hornhautgewebe ist,
**dadurch gekennzeichnet, dass** das Licht in dem Wellenlängenbereich und in zumindest einem der zwischen der Cornea (1) und der Netzhaut (9) liegenden Bereiche großteils absorbiert wird und sich der Wellenlängenbereich von etwa 1600 nm bis etwa 1700 nm erstreckt.

2. Hornhaut-Flap-Schnittgerät nach Anspruch 1, wobei die Laserstrahlungsquelle ein Co:MgF2- oder Cr:YAG-Laser ist.

## Claims

1. Apparatus for cutting a flap in the cornea, said apparatus generating a cut in the cornea (1) and comprising a laser radiation source generating laser pulses in the femto-second range to generate a cut in the cornea (1),
wherein the light emitted by the laser radiation source is in such a wavelength range that the radiation causes a reaction in the cornea (1) of an eye,
wherein the reaction caused by the light in the cornea is a laser-induced optical brake-through of corneal tissue,
**characterized in that** the light in the wavelength range is absorbed in at least one of the areas in the direction of the retina (9) before the retina to a large extend and is in a wavelength range from about 1600 nm to about 1700 nm.

2. Apparatus for cutting a flap in the cornea according to claim 1 wherein the laser radiation source is a Co:MgF2-laser or Cr:YAG-laser.

## Revendications

1. Appareil de découpe du volet cornéen effectuant une coupe dans la cornée (1), comprenant une source de rayonnement laser émettant des impulsions laser femtoseconde pour effectuer une coupe dans la cornée (1), la lumière émise par la source de rayonnement laser étant située dans une telle plage de longueurs d'onde qu'elle engendre une réaction dans la cornée (1) d'un oeil,
la réaction engendrée par la lumière dans la cornée (1) se traduisant par une percée optique du tissu cornéen induite par laser,
**caractérisé en ce que** la lumière est en grande partie absorbée dans la plage de longueurs d'onde et dans au moins une zone située entre la cornée (1) et la rétine (9) et **en ce que** la plage de longueurs d'onde s'étend d'environ 1600 nm à environ 1700 nm.

2. Appareil de découpe du volet cornéen selon la revendication 1, la source de rayonnement laser étant constitué par un laser Co:MgF2 ou un laser Cr:YAG.
